# EUROPEAN PATENT SPECIFICATION

(11) **EP 0 991 944 B1**
(45) Date of publication and mention of the grant of the patent: **25.08.2004**
(21) Application number: 98938647.9
(22) Date of filing: 25.06.1998
(51) Int. Cl.: G01N 33/547, C07K 17/06, C12N 11/06, C07K 17/00

(54) **METHODS FOR COVALENT IMMOBILISATION OF BIOMOLECULES TO A CARRIER BY MEANS OF A HIS-TAG**
VERFAHREN ZUR KOVALENTEN IMMOBILISIERUNG VON BIOMOLEKÜLEN AN EINEM TRÄGER MITTELS EINES HIS-TAGS
PROCEDES POUR L'IMMOBILISATION DE BIOMOLECULES PAR COVALENCE SUR UN VECTEUR A L'AIDE D'UN MARQUEUR D'HISTIDINE

(30) Priority: 25.06.1997 EP 97870095
(43) Date of publication of application: 12.04.2000
(73) Proprietor: N.V. INNOGENETICS S.A., 9052 Gent (BE)
(72) Inventor: BOSMAN, Alfons, B-1745 Opwijk (BE); VAN WIJNENDAELE, Frans, B-9270 Kalken-Laarne (BE); VAN DEN BROECK, Dirk, B-9890 Gavere (BE); VAN DE VOORDE, André, B-9160 Lokeren (BE)
(86) International application number: PCT/EP1998/003883
(87) International publication number: WO 1999/000670

(56) References cited:
- WO-A-96/19730
- WILLIAMS ET AL.: "Covalent immobilization of protein monolayers for biosensor applications" BIOSENSORS & BIOELECTRONICS, vol. 9, 1994, pages 159-167, XP002050231 cited in the application
- O'SHANNESSY ET AL.: "Detection and quantitation of hexa-histidine-tagged recombinant proteins on western blots and by a surface plasmon resonance biosensor technique" ANALYTICAL BIOCHEMISTRY, vol. 229, 1995, pages 119-124, XP002050232 cited in the application

## Description

The present invention relates to methods for covalent immobilisation of biomolecules to carriers and membranes by means of covalent binding with fused histidine tags. The invention also relates to ways for increasing the probability of covalent binding between carrier or membrane and the histidine residues that make up the His-tag, by chosing the appropriate reactive groups and/or physico-chemical parameters. The present invention also aims at simultaneously providing a universal detection method with increased sensitivity after crosslinking.

Recombinant proteins are often expressed as fusion proteins in order to increase their expression level and/or to facilitate their purification. The fusion part may contain a chemical cleaving site (e.g. hydroxyl amine, CNBr) or an enzymatic cleaving site (e.g. enterokinase, thrombin) allowing the subsequent purification of the mature protein.

The followed strategies for purification of such fusion proteins may be based on enhanced binding to ion exchange chromatography resins by introducing a polycationic (poly Lys , poly Arg) or a polyanionic tail (poly Asp, poly Glu). Most fusion proteins were developed in order to introduce an affinity chromatography step, because this is more specific and allows a higher enrichment during chromatography. Examples of these affinity based fusion partners are: prot A for purification on immobilised lgG (Löwenadler et al, EMBO J, 5, 2393, 1986), GST fusion for purification on immobilised GSH (Smith & Johnson, Gene, ***67***, 31,1988) or maltose binding protein for purification on immobilised maltose (Chu Di Guan, Gene, ***67***, 21, 1988). A further refinement of the latter approach has been the introduction of small affinity tags: e.g. streptag for binding on aminobiotin ( Schmidt &Skerra, Prot. Engineering, ***6***, 109, 1993), the Z domain of Protein A ( Murby et al, Biotechnol. Appl. Biochem., ***14***, 336, 1991), the mannose binding domain ( "Taylor &Drickamer, Biochem.J, ***274***, 575, 1991) or the (His)₆ tag. Histidine-tags are for the moment extremely popular in biotechnology. They allow easy purification on immobilized metal affinity chromatography (IMAC). The 'IMAC' purification concept was introduced by Porath ( Nature, 258, 598-299, 1975 ). He found that some amino acids like histidine, tyrosine and cysteine may interact on a non-covalent (electron donor- acceptor) basis with metal ions and he applied this finding for protein purification by immobilising the metal ions through a resin bound chelator like iminodiacetic acid (IDA). IMAC has the advantage that the binding between the metal ion and the protein is easily broken by a competitive agent like imidazole or by pH lowering and that the resin can be regenerated by an EDTA wash. The protein- metal ion interaction is not influenced by the presence of non-ionic detergents or high salt concentrations.
The strenght and specificity of the protein- metal ion interaction is dependent on the topographical distribution of the histidine residues ( Hemdan et al., PNAS, ***86***, 1811-1815, 1989 ).

The applications of IMAC in biotechnology were increased enormously by the introduction of the (His)₆ tags in recombinant fusion proteins and the use of nitrilo triacetic acid (NTA) as resin immobilised chelator (Hochuli *et al.,* Bio/Technology, ***6***, 1321-1325, 1988). IMAC can be applied in non-denaturing as well as denaturing (6M ureum, 6M Gu.HCl) conditions and an over 95% pure recombinant protein can be obtained in a single chromatography step. Furthermore, the use of (His)₆ tag is favoured by the fact that the tag does not interfere in most cases with the biological activity or the immunological reactivity and that the (his)₆ sequence is less toxic for *E. coli* than the other tested tag sequences (Viaplana & Villaverde, ***12***, 723-727, 1996). Raising antibodies against the (His)₆- tag is rather difficult, indicating that this sequence has only low immunogenicity. Other metal binding sequences were published ( Ljungquist *et al., **186,*** 563-569, 1989; Smith *et al,* J Biol. Chem., **263,** 7211-7215, 1988) but these sequences did not reach the popularity of the (His)₆ tag.
The IMAC purification concept has been transferred to polysterene plates for ELISA applications (Paborsky *et al,* Anal. Biochem., **234,** 60-65, 1996 ) and biotinylated NTA has been used for the detection of (His)-tag fusion proteins on Western Blot ( O' Shannessy *et al*,Anal.Biochem.J. ***229***, 119-124, 1995) The IMAC concept has also been applied successfully for reversible immobilisation on IDA resins for glycoproteins after potassium periodate oxidation and histidine coupling (Chaga, Biotechnol. Appl. Biochem, ***20***, 43-53,1994 ). The tripartite chelator- metal ion - (His) tag interaction has also been used for insertion of fusion proteins in liposomes ( Dietrich *et al,* Biochemistry, ***35***, 1100-1105, 1996) and immobilisation of nucleic acids on carriers ( Komissarov *et al* , Anal. Biochem, ***247***, 2106-2113,1997). Recombinant proteins have been reversibly (non covalently) immobilised by their histidine tags to metal- chelators which on their turn were covalently bound to microtiterplates (Paborsky *et al,* Anal. Biochem, 234, 60-65, 1996), to synthetic lipids (Dietrich et al, Biochemistry, 35, 1100-05,1996) or to the biacore biosensor membrane ( Gershon & Khilko,J Immunol Methods, ***183***, 65-76, 1995). Glycoproteins were complexed to metal affinity resin after oxidation and coupling of histidine. The resin was easily reused after regeneration with EDTA ( Chaga, Biotechnol Appl Biochem, ***20***, 43-53, 1994).

While His-tags, fused to recombinant proteins are extremely efficient for purification protocols they present one important drawback when used for immobilising biomolecules to carriers or membranes. The IDA (or NTA) - His-tag interaction is non-covalent and thus, by definition reversible, and dependent on equilibrium rules. This results in negative effects like off-binding, loss of material, and instability of the product, product migration ('band shuffling') and this in turn results in a lower sensitivity or incorrect conclusions. For this reason covalent attachment of biomolecules to membranes or carriers often seems a better approach. Biomolecules can be attached to membranes or carriers through reaction with reactive groups that often have a broad spectrum, thereby leading to a more or less stochastic immobilisation of the biomolecules, often resulting in a severe loss of bioactivity. This has been prevented in the past by providing the biomolecules with a tail consisting of chemical groups with a higher reactivity that simultaneously acts as a spacer. The frequency of inactivation upon immobilisation of the biomolecules thereby decreases drastically. For recombinant proteins, these tails can easily be attached by translational fusions with a stretch of highly reactive amino acid residues. Poly Lys, poly Arg, poly Glu, poly Asp and poly Cys have been considered and applied in the past, poly Lys with the highest success. Though histidine residues are also fairly reactive, poly His tails have never been considered as a solution to the above problem. Histidine residues are, together with α- and ε-amino groups, cysteine, tyrosine, arginine, methionine and acidic residues the most reactive residues. Histidine residues (pK~6.5) react readily with most acylating, alkylating and many electrophilic reagents. Photo-oxidation has also been used for the covalent immobilisation of proteins by their histidine residues to a hydroxypropylmethacrylamide polymer (Shen et al., J. Photochem Photobiol, ***34***, 203-210, 1996) or to create protein cross-linking (Verwej *et al.,* Biochem Biophys Acta, *647*, 87-94, 1981). Covalent immobilisation of protein monolayers by linker attachment to a silicon nitride surface or protein was studied by Williams& Blanch (Biosens Bioelectron, ***9***, 159-167, 1994). However, although the frequency of histidine residues is rather low in proteins, they often play an important role for the biological activity of the protein and the imidazole groups are fairly reactive. This might partly explain the lack of interest to consider poly His tails as a means for covalent immobilisation.

On the other hand, because histidine residues are often important for the biological activity of proteins, they are well studied by means of specific chemical modifications. Chemical modification is defined as a process resulting in a covalent derivatisation of amino acid residues, preferentially in a selective and specific way and without altering the conformation of the protein. In this way, it can be established whether histidine residues play an important role in the enzymatic process of certain enzymes.

It is an aim of the present invention is to decrease the probability of loss of bioactivity of a biomolecule upon immobilisation.

It is also an aim of the present invention to provide new methods for covalent immobilisation of biomolecules to carriers or membranes.

It is also an aim of the present invention to provide new functionalities to His-tags that are currently used in purification protocols.

It is also an aim of the present invention to increase the stability of a product that results from the immobilisation of a biomolecule.

It is also an aim of the present invention to provide methods that can increase the binding and/or the covalent immobilisation of biomolecules by means of amino acid residues that make up a His-tag.

It is also an aim of the present invention to simultaneously provide a universal detection method for biomolecules that contain a His-tag.

The solution to the above technical problem is achieved by providing the embodiments characterized in the claims and set out below. The present invention relates to a method wherein the presence of His-tags is exploited for covalent immobilisation of a biomolecule that contains said His-tag, and wherein the amino acid residues that comprise said His-tag are directly involved in the covalent bond. This His-tag, being part of the molecule, significantly increases the probability of immobilising the biomolecule in an oriented way without disturbing the conformation and functionality of the biomolecule. The probability of specifically linking the biomolecule by direct reaction of the histidine residues that are comprised within said His-tag (and thus not by histidine residues of the protein itself) is already enhanced by the fact that (1) histidine residues in proteins are rare and are usually shielded under native conditions and (2) the histidines in the fusion tail are easily accessible, because the recombinant proteins are very often purified on IMAC under non denaturing conditions. The majority of polar amino acids (e.g. Lys, Arg, Gly, Asp) are located at the surface of the protein, while the hydrophobic (non- polar) amino acids are shielded from the water environment. A gradient in polarity is thus formed from the exterior to the center of a protein and this gradient can also be found in 'pocket-like' structures at the protein surface. The accessibility of the residues together with their microenvironment (solvent, residues in neighbourhood) are in addition to their nucleophilicity important aspects for the reactivity with modifying agents.

Preferentially, said His-tag is already present in the expression product (fusion protein), and it may be located aminoterminally or carboxyterminally or even internally of the recombinant protein. Said His-tags, however, may also be introduced by chemical modification, thereby anchoring said His-tag at any possible site of the biomolecule. Preferably said His-tags are located outside of the protein at locations that are not interfering with the conformation of the protein upon immobilisation, and that preserve bioactivity when enzymatic reactions are aimed for and/or immunoreaction when antigen-antibody interactions are aimed for. At the same time the His-tag functions simultaneously as a spacer in order to improve the presentation, so that interaction with the support or carrier molecule is minimized.

It has to be understood that whenever the term 'His-tag' is used throughout the specification, a broad definition can be given to it, meaning any sequence that has an amount of histidine residues that is sufficiently high to allow appropriate application. In principle a 10 amino acids long sequence that has two histidine residues, and is repeated at least twice should suffice. More typical, said 'His-tag' contains at least three histidines within a stretch of 6 amino acids. The term 'His-tag' can also include the histidine rich domain of the WHP protein of E.coli (Wülfing et al., J.Biol. chem., ***269***, 2895, 1994), or any repetition of a histidine immediately followed by at least one residue. Preferably the term His-tag refers to at least three consecutive histidine residues, more preferably to at least four histidine residues, or even more preferably to at least five or at least six consecutive histidine residues.

The term 'biomolecule' as used throughout the specification refers to any molecule of biological origin including DNA, RNA, proteins, peptides, amino acids, metabolic compounds, sugars, lipids, and the like, and combinations thereof, that can be used for diagnostic and therapeutic purposes. The term 'biomolecule' thus refers to such molecules that have been obtained through purification from living systems. The term 'biomolecule' also refers to molecules that have been synthesised chemically or that have been obtained by recombinant means, or combinations thereof, thereby mimicking molecules as obtained in living systems without seriously altering their biological activity or function, thereby including peptidic nucleic acids (PNA's), drugs that can mimick certain substrates or hormones, or that mimick epitopes, thereby interfering and/or binding with enzymes, receptors and antibodies respectively.

According to another embodiment, the present invention also relates to a method wherein said covalent immobilisation or covalent cross-linking is achieved by exploiting the presence of a His-tag and wherein a covalent bond is established between said carrier or membrane and the amino terminal free amine group or the carboxyterminal free carboxyl group of said His-tag.

According to another embodiment of the present invention a protein or peptide or any other biomolecule that contains a His-tag can be immobilised by means of said His-tag by using any kind of reagent that can react with histidine residues without having a particular selectivity for histidine. Reagents for histidine residue modification can be classified in 2 groups: non- specific reagents or reasonable histidine specific reagents. Although the list may be far from complete, the following reagents can be cited as non- Histidine specific reagents: alfa-haloacids and amides, bis-alkylhalides like 1,3 dibromoacetone, N-bromosuccinimide, Woodward K reagent, diazonium salts, styrene and styrene oxide, aldehyde, iodine, sulfonyl halides, dansyl cloride, cyanation , 1-fluoro- 2,4-dinitrobenzene, N-ethymaleimide. It has to be understood that other reagents not cited within this list, known to the man skilled in the art, are hereby included. This embodiment relates to the exploitation of the presence of His-tags within any kind of biomolecule to increase the probability of reaction with amino acid residues that comprise said His-tag, by increasing the number of histidine residues and/or improving the presentation of said His-tag towards the reactive groups.

According to a further embodiment the present invention relates to the manipulation of one or more physico-chemical parameters of the reaction conditions, thereby increasing the selectivity of the reaction towards the histidine residues that comprise the His-tag. The reaction pattern of the above-listed reagents with protein can be markedly affected by the reaction conditions (e.g. pH, temperature, solvent and/ or buffer used, degree of illumination, excess of reagent, and other physico-chemical parameters): i.e. the specificity of these reagents can be significantly increased to a reactive group by adaptation of the conditions. Nucleophilic substitution reactions of alkyl halides may be taken as an example for non-specific histidine reagents. The reaction occurs by a 'bimolecular mechanism': an alfa- carbonylgroup, like that of haloacetates, enhances the reactivity by direct displacement. The reactivity of the haloacetates depends on the halide (i.e. I > Br > Cl > > F), so that iodo- and bromoacetates are the more useful agents for protein modification. The relative reactivity of alfa- haloacetates towards protein functionalities is sulfydryl > imidazolyl > thioether > amine, but the pH can here be used as reaction moderator, because the nucleophilicity of the side chains strongly differs ( pKₐ of -SH ~ 8.8-9.0; pKₐ imidazolyl nitrogen ~ 6.0- 6.5; pKₐ Lysine's epsilon amine ~ 9.5 and pKₐ alfa amino group ~7.5-8.0). Iodoacetate thus reacts optimally with sulphydryl groups at ~ pH 8. At pH ~ 6, where imidazole groups are at least partially unprotonated, the modification of histidyl side chains is sufficiently fast to be the principal reaction in those proteins not having accessible sulphydryl groups. The reaction with imidazole groups can proceed in 2 steps to a stable 1,3 -dicarboxylmethyl derivative. The alfa-haloacetamide derivatives have the same trend of relative reactivities and will be used typically as blocking agents, because the carboxylate functional group is absent. Diazonium groups contain reactive hydrogens that are able to participate in conjugation reactions and they are easily displaced by an attacking electrophylic group able to form a new stable covalent bond. The histidine side chains and the tyrosines are the targets of the diazonium salt. The pH can act here again as reaction specifier: at pH 8 the diazonium salts react principally with the histidyl residues ; at higher pH the phenolic side chains of tyrosine can also be modified.

According to another embodiment the interference of reactive groups in the biomolecule (predominantly Cys and Lys residues) may be excluded by a prior and reversible derivatisation of these groups (e. g. sulphonation of Cys, citraconylation of Lysines). The reversible modification groups are removed after the immobilisation or crosslinking of the protein. The interference of reactive histidines or cysteines in the active center may be circumvented by crosslinking or immobilising in the presence of a substrate or substrate derivative or competivive or non-competitive inhibitor, preferably reversible, thereby shielding the residues from reaction and the protein from inactivation, and possibly also preserving the native conformation of the protein or peptide or biomolecule during the immobilisation process.

According to another embodiment the present invention also relates to methods that can increase the probability of reaction with the histidine residues of the His-tag of the biomolecule, by combining a low pH during the reaction conditions and increasing the hydrophobicity of the membrane or carrier to which said biomolecule has to be immobilised covalently. The low pH, preferably less than 6, aims at increasing the hydrophobicity of said His-tag. At low pH conditions the imidazole groups of the histidine residues are fully deprotonated, leaving the protein with a fairly hydrophobic histidine rich tail at the outside of an otherwise fairly hydrophilic protein. When this is brought into contact with a hydrophilic-hydrophobic interphase, the His-tags will seak contact with the hydrophobic phase, leaving the rest of the protein within the hydropilic or aqueous phase. The hydrophobicity of the His-tag may also be enhanced by including hydrophobic residues. Increasing the hydrophobicity of the membrane or carrier thus aims at providing such interphase, wherein contact between the reactive groups present on said membrane or carrier with the histidine residues of the His-tag is increased, while contact with other reactive groups or single histidine residues that are part of the rest of the biomolecule is decreased, thereby driving the reaction towards covalent binding with the His-tag. The hydrophobicity of the carrier or membrane may be increased, simply by chosing the appropriate material of which said carrier or membrane is constituted. An example of this is the use of polystyrene. Alternatively, additional compounds may be integrated in or attached to the polymer that have a tendency to increase the hydrophobicity of the microenvironment of said membrane or carrier. Glass-beads or plates or nylonstrips can be silanised with 2-(3,4,epoxy-cyclohexyl)etyl trimethoxysilane. Neutral nylonstrips can be treated with hydrophobic silicons. Preferably with (epoxy cyclohexyl etyl) Methylsiloxane-Dimethylsiloxane copolymers. Such a silicon will coat the strip with a hydrophobic film favouring reaction with the histidines that comprise the His-tag, and still allowing reaction with ringstrained epoxy cyclohexyl groups. Alternatively, said carrier or membranes can be treated with compounds that due to their inherent amphoteric character will create an interphase at the carrier or membrane surface. Also the present invention relates to the use of apolar solvents in which the carrier or membrane can be immersed, before transfer to an aqeous phase wherein the reaction with the His-tag containing biomolecule occurs.

According to another embodiment, the present invention also entails the use of anti- (His-tag) antibodies or microproteins or of Ni chelators like NTA or IDA, or any other molecule that has affinity for histidine residues. These compounds have the property of having a high to very high affinity for said His-tag. When these compounds are covalently attached or otherwise immobilised to said carriers or membranes, they can thus be used to bring the His-tag in close proximity with said carrier or membrane, upon which a covalent linkage of any kind can be engaged, thereby increasing the probability that the His-tag is involved in this reaction.

According to a further embodiment the present invention more preferably relates to the use of reactive compounds that have a moderate to high selectivity for histidine residues, again increasing the probability that the His-tag is involved in the covalent immobilisation of the biomolecule. Diethylpyrocarbonate (DEP), and photo-oxidation in the presence of dyes like rose- bengal or methylene blue are often cited as histidine specific modification agents. Diethylpyrocarbonate has been used extensively for determination of the location of a histidine residue at the active site of enzymes ( Fruchter & Crestfield, J Biol.Chem, 242, 5807-5812, 1967; Davis *et al,* Biochemistry, ***33***, 1278-86, 1994;Kunno *et al,* Arch Biochem Biophys, ***277***, 211-7 1990 ) and cytokines as Tumor necrosis factor (Yamamoto *et al,* Protein engineering,***2***, 553-559, 1989 ) and recombinant human macrophage colony stimulating factor - β (Glocker *et al,* Biochemistry, **35**, 14625-14633, 1996) and receptor (Lacorazza et *al,* Neurochem Int, ***28***, 77-87, 1996). The modification of histidine residues can be followed by an increase in absorbance at 230 to 240 nm and as such be used to quantitate the number of modified residues (Roosemont, Anal. Biochem, ***88***, 314-320,1978;Melchior& Farhney, Biochemistry, 9, 251, 1970). The reaction of diethylpyrocarbonate can be reversed by incubation with hydroxyl amine hydrochloride at pH 7 or by incubation in alkaline solution ( Melchior and Fahrney, 1970). Raab (Z. Biol., ***39***, 524, 1900 ) first reported the lethal effects of visible light on microorganisms treated with dyes and it was later attributed to the oxidation of certain amino acids. Methylene blue and rose bengal are the most commonly used dyes. They mediate reaction between histidine residues and thus allow the formation of a covalent bond between His-tags. The mechanism of reaction is still not totally clear, but various studies suggest that the photo-oxidation appears to be mediated by photochemically generated singlet oxygen and/or other reactive oxygen species ( Henderson& Dougherty, Photochem Photobiol, ***55***, 145-157, 1992 ). In general the quantum yields were found to increase with increasing oxygen concentration (Webster et al., Anal Biochem, ***179***, 154-157,1989). The photo-oxydation proceeds rapidly even at low temperature and its specificity can again be influenced by the pH (Straight & Spikes, *Singlet O*_{*2*} Vol IV, CRC Press, Boca Raton, FL, pp 91-143, 1985 ). Rose bengal seems to be the most selective for histidine residues: the dye specificity is thought to be favored by the formation of short lived dye-imidazole complexes (Bellin and Yankus, Arch. Biochem. Biophys., ***123***, 18, 1968). Unprotonated imidazole groups of histidine react faster with the single oxygen than does the protonated form (Shen *et al.,* J. Photochem Photobiol, ***35***, 213-219, 1996). When this type of reaction is used for covalent immobilisation of biomolecules with a His-tag to a carrier or membrane, said carrier or membrane is first prepared by introducing histidine residues or His-tags or any other molecules that contain imidazole groups, such as histamine. This can be done by any reaction known in the art. Typically a poly-histidine sequence will be attached to said carriers or membranes through reaction with activated aldehyde, thereby creating a covalent bond between carrier or membrane and the end-standing aminogroup of the poly-histidine sequence. Histidine modification also occurs *in vivo:* quinone of acetylated catecholamines undergoes nucleophilic addition reactions with histidine in cuticular proteins ( Xu *et al,* Arch Biochem Biophys, ***329***, 56-64, 1996); Michael addition types of alkanal adducts are present in proteins due to oxidative stress and are markers for diseases ( Friguet *et al,* J Biol Chem, ***34***, 21639- 21643, 1994; Bruenner *et al,* Rapid Commun Mass Spectrom, ***8***, 509-512, 1994; Ohya, Biol Pharm Bull, ***17***, 554-556, 1994). Bromobenzene-3,4-oxide alkylates histidine residues of rat liver proteins (Bambal & Hanzlik, Chem. Res Toxicol, ***8***, 729-35, 1995).

According to another embodiment, the present invention also relates to the use of the His-tag as a marker or detection tool, e.g. direct incorporation of labelled amino acid analogs during translation or chemical synthesis, or as tag for modification. Detection of histidines under specific conditions or by using specific reagents ( Nakamura, J Chromatogr, ***131***, 215-222, 1977; Sundler & Hakanson, Brain Res Bul1, ***9***, 107-116, 982) or by crosslinking the his-tag with binding compounds, like antibodies. This method has the advantage that the detection is universal and that the signal intensity is immediately correlated with the concentration of 'His-rich' sequence and no other tools/ modifications are necessary.

Furthermore, the 'histidine- rich' sequence can be used as a cross-linking element and the crosslinked biomolecules can then be presented for conjugation to the membrane/ support. This embodiment also relates to the use of branched peptides that can increase the formation of aggregates, or reactive centers for covalent immobilisation of biomolecules to the carrier or membrane. The latter would result in an enhanced concentration of antigen on the membrane and increase the sensitivity. The 'crosslinking' aspect may also be useful for raising antibodies against molecules, which have only weak immunogenicity. This effect may be compared with e.g. the already well described use of 1-fluoro- 2,4 dinitrofluorobenzene for crosslinking to carrier supports by lysines.

The term 'carrier' as used throughout the specification can be interpreted in a narrow way, meaning a solid carrier that allows for immobilisation of biomolecules onto a solid surface, thereby providing every advantage of immobilisation as known in the art. In this respect a solid carrier can be a membrane that can be used for providing a diagnostic or therapeutic tool. Said carrier, however, can also have any form that can be advantageous to the man skilled in the art, such as beads, microparticles, fibers, and hydrogels. A nonlimiting list of such solid carriers are nylon strips, polystyrene membranes, membranes based on polysaccharides such as cellulose and agarose, synthetic or biological based polymers, polyphosphazenes; and any silicon-based support. Such carrier, however can also be in the form of beads or microparticles that can be kept in suspension, or stacked in columns, or that can be used for precipitation. Such form can be advantageous for any form of biofermentation or any other process wherein the bioactivity of the immobilised biomolecule is aimed. It can also be advantageous as a step in a diagnostic method, for instance to provide a concentration step by means of precipitation. In this respect, the carrier or microparticles can be used to increase the sensitivity. A list of possible materials that can be used in this respect are other proteins or peptides or aggregates thereof, microparticles of viral origin, proteinaceous particles, or microparticles comprised of polysaccharides, DNA, RNA, oligonucleotides, or liposomes, or combinations thereof.

The term 'carrier' as used throughout the specification, may also be interpreted in a broad way. In this respect the carrier allows for covalent linkage with a biomolecule without resulting in a solid form, such as membranes or beads, that can be precipitated. The beads or microparticles can be that small that they will no longer result in a suspension that is precipitable, but will result in a colloidal solution or a solution. Such microparticles thus can be comprised of any molecule or molecules that allow for a covalent crosslinking with biomolecules by means of amino acid residues of the His-tag of said biomolecules, and that are small enough that they will not precipitate. However, through a process of crosslinking between such particles a process such as precipitation can still be engaged, providing a tool for e.g. diagnostic purposes. When the term 'carrier' is interpreted in this broad way, the present invention also relates to a method for covalent linkage between two biomolecules of which at least one contains a His-tag. These biomolecules can have a natural tendency of interacting with each other in a non-covalent way. This invention therefore also relates to a method for covalent stabilisation of oligomers. Examples of such oligomers are the dimers formed between two leucine zipper proteins or between Myc and Max proteins. In a typical situation both partners of such dimers are provided with a His-tag, e.g. aminoterminal of the leucine zipper. Upon oligomerisation, both His-tags are close enough to allow covalent linkage e.g. by means of Rose Bengal photo-oxidative crosslinking. One of the partners may also contain a sugar moiety that upon oxidation provides aldehyde groups that allow for covalent linkage with the histidine residues of the His-tag.

It has to be understood that the invention also relates to any combination of the methods as set out by the different embodiments.

It also has to be understood that the invention also relates to any product that has been obtained by any of the embodiments as set out above, and the use thereof.

The use of immobilized enzymes and particularly of enzymes which have been rendered insoluble has gained increasing importance in the industrial processes, e.g. the fermentation industry, in the production of antibiotics, and in chemical and/or biological syntheses. Additional fields of application include but are not limited to metabolic investigations, especially for diagnostic purposes wherein antigens are immobilised, and the use as medicines, specifically upon topical, but also with oral and parenteral administration, where the fixed biomolecules are preferably administered in the form of microcapsules. Within this field, one should also consider the increasing importance of the use of oligomers for instance in the form of bispecific antibodies with increased avidity, or oligomers with a enzymatic property on one hand and a targeting function on the other hand.

Because more and more of these biomolecules are obtained by recombinant techniques many proteins and peptides can be readily obtained in a one-step purification process by means of a fused His-tag that allows for metal immunoaffinity chromatography. The present invention demonstrates that the presence of such His-tags can be exploited directly for covalent immobilisation, a possibility that has been neglected for more than one decade. Such strategy allows for oriented covalent immobilisations by means of covalent bonds of readily available biomolecules, thereby decreasing the probality of inactivation of the biomolecule upon immobilisation. The probability of inactivation is further decreased because the His-tag functions as a spacer. Such form of immobilisation will also optimise the presentation of the biomolecule when interactions with other biomolecules are aimed for, as is the case for DNA-DNA interactions, DNA-RNA interactions, DNA or RNA-protein interactions and epitope-antibody interactions. At the same time, the His-tag, whether introduced chemically or by recombinant means provides a universal tag for detection by use of anti-His antibodies and/or microproteins. When introduced chemically, fluoresent dyes can be incorporated. The present invention also allows for covalent cross-linking between two biomolecules by means of a His-tag. This allows for the synthesis of highly stabile oligomers which can be extremely useful for diagnostics and therapy as is the case for bispecific antibodies. On top of this, the poly-histidine sequences have a very low immunogenicity which makes them especially suitable for therapeutic uses.

These and other uses of the invention, are intended to be within the scope of the invention. The examples below are enclosed only for illustrative purposes and should not be considered to limit the current invention in any way.

### Example 1: Crosslinking of (His)₆proteins in the presence of Rose Bengal in solution

a) *(His)*_{*6*} *p24 as antigen:* A 10 mg/ mL p24 (His) solution was dissolved in air saturated phosphate buffer at pH 7 and rose bengal was added to a final concentration of 0.01%. The solution is thermostated at 25°C and illuminated with a 300 Watt spotlight for 60 minutes. After the irradiation the sample is kept in the dark and the Rose Bengal dye is removed by dialysis against the phosphate buffer.
b) *(His)*_{*6*} *ScFv antibodies:* Anti IFN -g ScFv D9D 10(His) is purified by Ni²⁺ -IMAC and desalted on Sephadex G25 to 50 mM MES, 150 mM NaCl, pH 6.5. The ScFv crosslinking is performed as described in a). The crosslinked oligomeric scFv D9D10 are separated from the monomeric and rose Bengal by chromatogaphy in PBS on Superdex G75.

### Example 2: Immobilisation of His-tagged peptides on polymers

a) *Covalent binding by the His-rich sequence wherein the His-tag functions as spacer:*
   Non preactivated nylon membranes are incubated with 2.9 M HCl for 60 min at 40 °C and the membranes are consecutively rinsed with water and 0.1 M K₂HPO₄, pH 8.
   The membranes are incubated with 5 % (v/v) glutaraldehyde in 100 mM K₂HPO₄, pH 8.0 at 40°C for 60 min. The membranes are again washed with water and 50 mM phospatebuffer, pH 6.5.The peptide 315 (contains a C-terminal his-tag) is incubated overnight with the aldehyde activated membrane in 50 mM phospate, at pH6.5 and the non reactive groups are blocked after washing with 100 mM Tris-HCl, pH 8.0.
b) *Non- covalent binding on non activated microspheres after covalent crosslinking by the His-tags.*
   Peptide 315 (5 mg/mL) is crosslinked by the method as described in example 1.The crosslinked material is separated from the rose Bengal and monomeric peptide by gel filtration in PBS and the oligomeric fractions are mixed after concentration to 1 mg/ml with 10% microspheres (polysterene K030, Prolabo), which have been pretreated as described by the manufacturer.
   The solution of antigen- micoparticles complexes is centrifugated after overnight storage at 4°C and washed with PBS.The microparticles are blocked afterwards by incubation in buffer containing 2% gelatin.
   The covalent cross-linking increases the antigen density as well as the strength of binding on the microparticles.

### Example 3: Covalent Immobilisation of peptide by a histidine tag on preactivated membranes by including a supplementary spacer for use in LIA applications.

Amino activated nylon membranes (Pall Biodyne A) are treated with the heterobifunctional 250 µg/mL sulfosuccinymidyl (4-iodoactate) aminobenzoate (sulfoSIAB, Pierce) in 100 mM phosphate, 150 mM NaCl, pH 7.5 for 1 h at room temperature. The membrane is washed with the water and 100 µg/mL peptide 315 in 25 mM phosphate, buffer at pH 6.2 is sprayed on the activated membrane.
The non reacted active groups are blocked after washing with an excess of cysteine in 100 mM carbonate buffer, pH 8.2 and the membrane is incubated with human serum n° IG 17 758 in order to verify the reaction with the antigen.
Detection is done by incubating the strip with alkaline phosphatase labelled antihuman lgG in Tris- buffer, pH 9.5 in the presence of NBT/BCIP and the staining reaction is stopped by addition of 10 mM EDTA.

### Example 4: Covalent Immobilisation of protein by a histidine rich tag on preactivated polymers by including a supplementary spacer for ELISA aplications.

Thiol-activated microtiterplates, obtained by modification of amino-activated microtiterplates, are incubated in the dark with the bifunctional bis-haloacetyl dibromoacetone and the excess of reagent is removed by washing the polystyrene plates. Purified sulfonated H6TPN15 (5 *µ*g/mL) is incubated for 2h in 50 mM phosphate buffer, pH 6.3 and afterwards washed with the same buffer.
The left reactive groups are blocked with 5 mM Cys in phosphate buffer, pH 7.5 and the well is incubated with human serum n° IG 12537 in order to verify the reaction with the antigen.
Detection is done by incubating with horse peroxidase labelled anti-humanlgG in in the presence of TMB and the staining reaction is stopped by addition of 1N sulfuric acid.

### Example 5: 'His' tag mediated oriented covalent immobilisation in the presence of a 'His' tag complexing agent.

Purified Anti-(His)₆ antibodies (1 mg/mL in Borate buffer, pH 9.0) are covalently linked to MiniLeak resin (Kem En -Tec, Denmark). The nonreacted antibodies are removed by washing and the left active groups are blocked by incubation in 100 mM ethanolamine, pH 9. Purified HCV E1s.His (0.1 mg/ L) is mixed with the resin and the free E1s(His) is removed by washing with PBS. The E1s (His)- anti(His) antibody complex is covalently crosslinked by addition of 5 mM sulfoBSOCOES in borate buffer pH 9.0. Non covalent bound antigen is removed by washing the well with 100 mM glycine-HCl.
The crosslinked antigen- antibody column is used for the purification of E1s binding proteins like receptor.
Alternatively, the antibody- antigen crosslinking and the separation of free antigen is performed before the presentation to the resin.

### Example 6 :His tag mediated oriented covalent immobilisation of monoclonal antibodies for immuno affininity purification.

The anti- Interferon- g monoclonal antibody F1 is treated with periodate to oxidize the glycidic moieties to aldehyde . After removal excess reagent, the modified antibody is incubated at pH 6.5 with poly (His). The unreacted poly (His) is removed by gel filtration and the poly(His)- modified MAb is added at pH 6.5 to a polysaccharide carrier, which has been activated in advance by periodate treatment.
CHO medium containing interferon gamma is loaded on the antibody column and the resin is washed with a 25 mM phosphate buffer, pH 7,5, 150 mM NaCl. The IFN-g is eluted by applying 100 mM trisodium phosphate buffer, pH 11 and the eluate is neutralised immediately by addition of 1 M acetic acid.

### Example 7: Antigen oligomerisation by covalent crosslinking for immunisation and antibody production.

a) The tetrabranched peptide 315 with a (his)₆ tag as a starting sequence is solubilized in MES buffer, pH 6.5 and incubated in the dark in the presence of 0.01 % rose Bengal. The photooxidation is performed as described in example 1. The crosslinked forms of the peptide are separated by gel filtration on Superdex G75 in PBS and injected into animals (e.g. rabbits for polyclonal antibodies, mice for monoclonal antibodies) after mixing with complete Freund's adjuvans (CFA).
b) alternatively, the Rose Bengal is replaced by the bifunctional reagent 1,3 dibromoacetone for crosslinking and the crosslinking is performed in a MES buffer pH 6.5. The crosslinked antigens are separated by gel filtration in PBS and after mixing with the CFA injected into animals.

### Example 8: Elimination of interfering reactive groups before covalent immobilisation of the biomolecule by the histidine- rich sequence.

*a) Reversible blocking of the enzymatic active center*
   The purified papaine(His)₆ protein is modified by treatment with a 70 fold excess of methylmethanethiosulphonate (versus thiol of papain) in phosphate buffer, pH 7.0 at 4°C for 4 h in order to modify the cysteine in the active center of the thiol enzyme.
   The solution is acidified to pH 4.5 and the excess of blocking reagent is removed by gel filtration on Sephadex G25. The papaine (His) fusion protein is immobilised in 50 mM MES buffer, pH 6.5 by incubating a polysaccharide support that has been activated in advance with periodate.
   The immobilized enzyme is reactivated by treatment in 35mM Tris- HCl buffer, pH 8 containing an excess of a thiol compound e. g. 10 mM cysteine in order to remove the blocking agent.
*b) Elimination of interfering groups on antigens*
   Purified recombinant TPNp15(His) fusion protein is solubilized in 25 mM Borate-HCl, 6M ureum, pH 8.5, 2mM DTT. Citraconic anhydride is added gradually over 1 h under continuous stirring to the solution till a 100 molar excess of lysines in the protein is obtained. The pH is maintained with 1N NaOH at pH 8.5 by using a pH titrator. After 2 h of incubation at room temperature, solid sodium phosphate is added till a final concentration of 50 mM is reached and the pH is adjusted to pH 7 with 2 N HCl. Solid Na₂S₄O₆ and Na₂SO₃ are added under continuous stirring till a final concentration of 65 mM and 130 mM respectively and incubated for 6H at room temperature.The solution is desalted to PBS on Sephadex G25 and the protein solution is diluted to 100 *µg*/ mL in MES buffer, pH 6.5 just before spraying on the LIA membrane, which has been activated by glutaraldehyde as described in example 2a. The unreactive groups on the membrane are blocked with 50 mM ethanolamine buffer, pH 9.
   The citraconylated and sulphonation groups are removed by incubation of the membrane consecutively at pH 4 for 2 h and in 50 mM Tris-HCl, pH 8.5 containing 1 mM DTT and 1mM EDTA for 30 min.

### Example 9: Simultaneous antigen detection of different diseases based on the His tag.

a) The *E. coli* expressed antigens syphilis (His)₆ p15 , the HIV O (His)₆ p24 and the HCV (His)₆ E2s are purified by IMAC on IDA Sepharose FF and after desalting and dilution to a final concentration of 1 µg/ ml coated in bicarbonate buffer on the ELISA polystyrene plates.
On the other hand, HRP ( 10 mg/mL) is solubilized in 10 mM phosphate buffer, pH 7.2, 150 mM NaCl and oxidized in the dark for 30 min by treatment with 8 mM periodate. The aldehyde activated HRP is desalted in PBS and the pooled HRP fractions are incubated for 2 H at room temperature in 100 mM borate buffer pH 8.2 with a 2 fold molar excess of poly(His). The non reacted active groups are blocked with 50 mM ethanolamine, pH 8.5 and the enzyme is separated from the peptide by gel filtration in 50 mM MES buffer, pH 6.5, 150 mM NaCl. The HRP-peptide solution is mixed with an equimolar amount of each (His)₆ antigen and crosslinked by treatment with rose bengal as described in example 1. The covalently photocrosslinked antigen-HRP complexes are separated from the free (His)₆ antigen by chromatography on Con A Sepharose and the bound complexes are eluted by PBS containing 0.5 M a -Methylmannoside.
The human serum is diluted in the sample diluent buffer and after 2 h incubation at 37°C washed in order to remove the free serum proteins.
The HRP- antigen complex is used in bridging ELISA and TMB is used as detection reagent.

### Example 10: The binding of his containing oligo to activated nylon membrane

*a) covalent binding without crosslinking:*
   1.25 mg 1-ethyl-3-(3-dirnethylaminopropyl)carbodiimide.HCl (EDC.HCl) is weighed in a eppendorf tube.
   7.5µL oligonucleotide ( ~ 100 µg or ~ 10 nmol), containing a 5' phosphate group and 5µL of 0.25 M bis-hydrazide compound in 0.1 M imidazole, pH 6 are added and mixed.
   After 5 min an additional 20 µL of 0.1 M imidazole is added and the solution is incubated for 30 min. at room temperature.
   A 5 fold molar excess NH₂- (Gly)₂- (His)₄ peptide to the nucleic acid solution is added and incubated for 1h at 50°C.
   The probe is desalted on Sepdadex G25 in phosphate buffer, pH 7.2, 10 mM EDTA.
   The 'His'- labelled oligo is activated in the presence of EDC.HCl and presented to the Pal nylon Biodyne A membrane ( nylon -NH₂).
*b) The binding of (His)*_{*6*} *containing oligos to the membrane after crosslinking*
   The proces is identical as described in the previous example, until the binding of the His rich sequence to the oligo.
   The covalently oligo-His complex is desalted by Sephadex G25 in phosphate buffer, pH 7.0 and kept in the dark when rose bengal is added to the solution. The solution is saturated with oxygen, stirred and illuminated with a spotlight of 300 W for 30 min. The solution is kept in the dark and the products are separated from the rose bengal by chromatography on Sephadex G25 in phospate buffer, pH 7.0.

### Example 11:

Maleic anhydride activated nylonstrips ABC (LIA strips) are reacted at pH 9.0 with peptide 315 containing a high proportion of histidine ,tyrosine, and tryptophan dissolved in carbonatebuffer pH 9. After a 5 min. reaction the excess peptide is washed away with carbonatebuffer containing 5% DMSO.
The Ni²⁺- complex of recombinant antigen Ro60, containing a histidine tail is prepared by incubation of the protein in 6M Guanidine or ureum at a concentration of 2mg/ml with 5mM NiCl₂. Following a 5 minutes equilibration at room temperature the complex is dialysed against carbonate buffer or PBS, followed by dilution to 100 µg/ml which is the spraying concentration.
Right before spraying the solution is supplemented with 100 µM monoperoxyphalic acid (MMPP) and the strip left for five minutes at room temperature.
Alternatively the solution is sprayed without the addition of MMPP, but after spraying, the strip is incubated in 100*µ*m MMPP. Quenching of the strips is perfomed by reaction with Tris 0.1M buffer at pH 9.0.
The rest of the procedure is according to established LIA procedures such as for instance disclosed in WO 96/13590.

### Example 11b.

Nylonstrips are incubated with peptide 315 containing six terminal histidines. Consequently the membrane is incubated with Nickelchloride to saturate the Histidines. Excess NiCl₂ is washed away.
The membrane is sprayed with SmD antigen solution at the usual concentration. containing MMPP for crosslinking. Further development of membranes is as performed in the standard procedure described for LIA.

### Example 12.

Maleic acid anhydride activated nylonstrips ABC_are reacted with peptide 315 (with 6 C-terminal histidines) in carbonate buffer at pH 9 for 5 minutes and then washed with carbonatebuffer.After blocking the strip with 100 mM Tris-HCl, pH 8.5 and washing with water, the strip is incubated with 5 mM CrCl₃ or NiCl₂ at room temperature in water. The strpis are thoroughly washed to remove excess metal ions.

The strip is then incubated with oligonucleotide probe for detection of HIV resistance during in 6 x SSC during 60 minutes after which the strip is dried and blocked.
The PCR product is then applied in buffer containing 0.4 µM NaOH and 100 µM EDTA.
The hybridisation is performed as described for nitrocellulose membranes.

### Example 13.

Polyphosphazene microparticles are prepared that contain peptide 315 (histidine-tail, 6 H)
grafted on the polymer with which the microparticles are prepared and suspended in 0.1M sodium phosphatebuffer of pH 7.4 . Recombinant E1 HCV protein containing a histidine stretch dissolved in the same buffer containing 0.05% Chaps. The mixture is air saturated and Rose Bengal is added in a final concentration of 25 µM. The mixture is stirred for 2 hours at 25 °C in air and illuminated with broad-band light and with fixed light energy flux.
The reaction mixture is collected , de-aerated and excess non-reacted E1 and crosslinked E1 is separated from the E1 containing particles by size exclusion chromatography.
The particles as well as crosslinked E1 fractions were used for immunisation of mice and the immunostimulant activity of phosphazene was compared to complete Freund's adjuvans (CFA).
On the other hand , the particles are coated on ELISA plates for use in our E1 monitoring test.
The OD responses and performance of these particles are compared with E1s oligomers.

### Example 13b.

In a parallel experiment, E1 is used that does not contain a histidine tail.

### Example 13c

In another parallel experiment, the recombinant proteins E1 and NS3, which contain both a His tag sequence, are mixed in equimolar amounts and incubated in the solution with the particle in order to create a multifuntional detection system after crosslinking by rose bengal.

### Example 13d

Using same Polyphoshazene microparticles containing peptide 315 but using procedure as described in example 11 to couple histidine containing recombinant proteins.

### Example 14:

a) Glass strips (SiO₂ with silanols) are cleaned. For silanation with 2-(3,4-epoyecyclohexyl)ethyl-trimethoxy silane, the strips are placed in an inert atmosphere in 2% solution of the epoxy alkoxyde in dry toluene for a period of 2 hours. The strip is removed from the solution , rinced in dry toluene and allowed to airdry. A film of silane is applied in such way.
   The strips are then sprayed with His-tagged E1 or E2 envelope recombinant proteins dissolved in Phosphatebuffer pH 6,0 containing 0.1 % CHAPS.
   Residual epoxy groups are blocked afterwards by incubation in 1 % Ethanolamine for 2h at room temperature.
b) Maleic acid anhydride activated Nylonmembrane are reacted with 2-(3,4 - Epoxycyclohexyletyl) -Amino to create ringstrained epoxycyclohexyl groups. The membranes are incubated with His-tagged NS3 at pH 6.0 and the membranes are blocked by treatment with ethanolamine.

### Example 15:

a)The neutral nylon strip are treated with a 1 % solution of a hydrophobic silicon, preferably with epoxy-cyclohexyl-ethyl)methylsiloxane-dimethylsiloxane copolymer and dried at room temerature. His tagged NS3 is sprayed at 100 µg/ml in phosphate buffer, pH 6.0 and dried.
b) Amino activated nylon membranes are treated with an excess of the hydrophobic silicone as described in a).The further treatment with His-tagged proteins are as described in a.

## Claims

1. Method for covalent immobilisation and/or covalent conjugation of proteins or peptides or other biomolecules to a support or carrier by exploiting the presence of a His-tag, and wherein the covalent linkage involves amino acid residues of said His-tag, which can be located amino terminally or carboxy terminally or internally of said protein or peptide and is introduced by chemical or recombinant means.

2. Method according to claim 1 further **characterized in that** said His-tag comprises at least 3 histidine groups.

3. Method according to any of claims 1 to 2 wherein said covalent immobilisation and/or covalent conjugation is achieved by means of the amino terminal free amine group or of the carboxyterminal carboxyl group of the end standing amino acid residue of the His-tag.

4. Method according to any of claims 1 to 3 further **characterized in that** the reactive groups of said support or carrier are not particularly selective for histidine, but reactive groups of said support or carrier are not particularly selective for histidine, but wherein selectivity towards histidine is significantly augmented towards histidine over the other residues, by applying at least one of the following methods:
- using appropriate reaction conditions, such as pH, solvent, excess of reagent, kinetics, temperature and the like,
- fusing the His-tag to or into said peptide or protein or biomolecule to be immobilized in such way that said His-tag is located externally of said protein or peptide thereby facilitating interaction between the histidine residues of said peptide or protein and the reactive groups of the support or carrier,
- using a sufficient number of histidine residues to make up the His-tag wherein the said His-tag contains at least three histidines within a stretch of 6 amino acids, thereby increasing the probability of reaction between the reactive groups of the support or carrier and said histidine residues,
- by blocking other residues than histidine that could react with the reactive groups of said support or carrier,
- addition of competitive inhibitor or substrate, to block reaction of those reactive amino acids that make up the active centre,
- in combination with a lowering of the, pH, increasing the hydrophobicity of said support or carrier or of the micro environment of said support or carrier, thereby avoiding reaction with residues other than histidine and increasing interaction with amino acid residues of said His-tag, or
- bringing the His-tag in close proximity with said support or carrier through the use of anti His-tag antibodies or microproteins, or through Ni chelators such as NTA or IDA, or any other His-tag binding compounds.

5. Method according to any of claims 1 to 4 further **characterized in that** the reactive groups of said support or carrier have a selectivity for histidine, preferably wherein the reactive groups are chosen from the following groups:
- the use of rose bengal or of methylene blue to mediate photo-oxidative crosslinking between histidine residues that comprise the His-tag and histidine residues that are located on the carrier or membrane.
- the use of diethyl pyrocarbonate and derivative thereof.

6. Method according to any of claims 1 to 5 for covalent immobilisation and/or covalent conjugation of proteins or peptides or other biomolecules to a support or carrier by means of a His-tag wherein the activated groups of the support display selectivity towards the histidine residues and wherein the selectivity towards histidine residues of the His-tag is achieved through a combination of the methods as described in claims 1 to 5.

7. Method according to any of claims 1 to 3 wherein said His-tag of said peptide, protein or other biomolecule, is allowed to react with other His-tags, through photoreaction in the presence of oxygen and bengal rose or methylene blue or a bifunctional reagents, and wherein said other His-tag can be part of proteins, peptides or other biomolecules, or linked to a membrane or a carrier.

8. Method according to any of claims 1 to 7 wherein said support or carrier comprises:
- a membrane comprising membranes based on styrene or nylon,
- other proteins or peptides or aggregates thereof,
- microparticles of viral origin,
- microparticles of a polysaccharide nature,
- proteinaceous microparticles,
- biomolecules such as liposomes, DNA, RNA, oligonucleotides, liposomes.
- organic synthetic polymeric microparticles and biopolymeric microparticles, or
- inorganic substrates based on silica.

9. A method according to any of claims 1 to 8 wherein said His-tag comprises any kind of detectable label.

## Patentansprüche

1. Verfahren zur kovalenten Immobilisierung und/oder kovalenten Konjugation von Proteinen oder Peptiden oder anderen Biomolekülen an eine Stütze oder einen Träger durch Ausnutzen des Vorhandenseins eines His-Tags, und wobei an der kovalenten Verknüpfung Aminosäurereste des His-Tags, das am Aminoterminus oder Carboxyterminus oder im Inneren des Proteins oder Peptids lokalisert sein kann, beteiligt sind und die Verknüpfung durch chemische oder rekombinante Mittel eingeführt wird.

2. Verfahren nach Anspruch 1, weiterhin **dadurch gekennzeichnet, daß** das His-Tag mindestens 3 Histidingruppen umfaßt.

3. Verfahren nach einem der Ansprüche 1 bis 2, wobei die kovalente Immobilisierung und/oder kovalente Konjugation mittels der freien aminoterminalen Amingruppe oder der carboxyterminalen Carboxygruppe des endständigen Aminosäurerests des His-Tags erzielt wird.

4. Verfahren nach einem der Ansprüche 1 bis 3, weiterhin **dadurch gekennzeichnet, daß** die reaktiven Gruppen der Stütze oder des Trägers nicht besonders selektiv für Histidin sind, wobei jedoch die Selektivität gegenüber Histidin in signifikanter Weise zugunsten von Histidin gegenüber den anderen Resten durch Anwendung wenigstens einer der folgenden Methoden vergrößert wird:
- Verwenden geeigneter Reaktionsbedingungen, wie z.B. pH, Lösungsmittel, Reagenzüberschuß, Kinetik, Temperatur u.ä.,
- Fusionieren des His-Tags an oder in das zu immobilisierende Peptid oder Protein oder Biomolekül, so daß das His-Tag auf der Außenseite des Proteins oder Peptids lokalisiert ist, wodurch die Wechselwirkung zwischen den Histidinresten des Peptids oder Proteins und den reaktiven Gruppen der Stütze oder des Trägers erleichtert wird,
- Verwenden einer ausreichenden Anzahl an Histidinresten, aus denen sich das His-Tag zusammensetzt, wobei das His-Tag mindestens drei Sistidine innerhalb eines Abschnitts von 6 Aminosäuren enthält, wodurch die Wahrscheinlichkeit einer Reaktion zwischen den reaktiven Gruppen der Stütze oder des Trägers und den Histidinresten erhöht wird,
- durch Blockieren von von Histidin verschiedenen Resten, die mit den reaktiven Gruppen der Stütze oder des Trägers reagieren könnten,
- Zugabe eines kompetitiven Inhibitors oder Substrats zur Blockierung der Reaktion derjenigen reaktiven Aminosäuren, die das aktive Zentrum bilden,
- Erhöhen der Hydrophobizität der Stütze oder des Trägers oder der Mikroumgebung der Stütze oder des Trägers in Kombination mit einer Erniedrigung des pH-Werts, wodurch eine Reaktion mit von Histidin verschiedenen Resten vermieden und die Wechselwirkung mit Aminosäureresten des His-Tags erhöht wird, oder
- Positionieren des His-Tags in enge Nachbarschaft zu der Stütze oder dem Träger durch Verwendung von Anti-His-Tag-Antikörpern oder Mikroproteinen oder durch Ni-Chelatoren, wie z.B. NTA oder IDA, oder andere His-Tag-bindende Verbindungen.

5. Verfahren nach einem der Ansprüche 1 bis 4, weiterhin **dadurch gekennzeichnet, daß** die reaktiven Gruppen der Stütze oder des Trägers eine Selektivität für Histidin aufweisen, wobei die reaktiven Gruppen vorzugsweise aus den folgenden Gruppen ausgewählt sind:
- Verwendung von Bengalrosa oder Methylenblau zur Vermittlung einer photooxidativen Quervernetzung zwischen Histidinresten, die das His-Tag umfassen, und Histidinresten, die auf dem Träger oder der Membran lokalisiert sind.
- Verwendung von Diethylpyrocarbonat und einem Derivat davon.

6. Verfahren nach einem der Ansprüche 1 bis 5 zur kovalenten Immobilisierung und/oder kovalenten Konjugation von Proteinen oder Peptiden oder anderen Biomolekülen an eine Stütze oder einen Träger mittels eines His-Tags, wobei die aktivierten Gruppen der Stütze eine Selektivität gegenüber den Histidinresten zeigen und wobei die Selektivität gegenüber Histidinresten des His-Tags durch eine Kombination der Verfahren, wie sie in Ansprüchen 1 bis 5 beschrieben sind, erreicht wird.

7. Verfahren nach einem der Ansprüche 1 bis 3, wobei man das His-Tag des Peptids, Proteins oder anderen Biomoleküls durch Photoreaktion in Gegenwart von Sauerstoff und Bengalrosa oder Methylenblau oder einem bifunktionellen Reagenz mit anderen His-Tags reagieren läßt und wobei das andere His-Tag ein Teil von Proteinen, Peptiden oder anderen Biomolekülen sein kann oder mit einer Membran oder einem Träger verknüpft sein kann.

8. Verfahren nach einem der Ansprüche 1 bis 7, wobei die Stütze oder der Träger:
- eine Membran mit Membranen auf Styrol- oder Nylonbasis,
- andere Proteine oder Peptide oder Aggregate davon,
- Mikropartikel viralen Ursprungs,
- polysaccharidartige Mikropartikel,
- proteinartige Mikropartikel,
- Biomoleküle, wie z.B. Liposomen, DNA, RNA, Oligonukleotide, Liposomen,
- organische synthetische Polymermikropartikel und Biopolymermikropartikel oder
- anorganische Substrate auf Siliziumdioxidbasis umfaßt.

9. Verfahren nach einem der Ansprüche 1 bis 8, wobei das His-Tag eine beliebige Art einer nachweisbaren Markierung enthält.

## Revendications

1. Procédé pour immobiliser par covalence et/ou pour conjuguer par covalence des protéines, des peptides ou d'autres biomolécules sur un support ou sur un transporteur, en exploitant la présence d'une étiquette d'histidine, et dans lequel la liaison covalente implique des résidus d'acides aminés de ladite étiquette d'histidine, qui peut être située sur ladite protéine ou ledit peptide de manière amino-terminale ou carboxy-terminale ou être à l'intérieur de ceux-ci et qui est introduite par voie chimique ou recombinante.

2. Procédé selon la revendication 1, **caractérisé en outre en ce que** ladite étiquette d'histidine comprend au moins 3 groupes d'histidines.

3. Procédé selon l'une quelconque des revendications 1 à 2, dans lequel ladite immobilisation covalente et/ou ladite conjugaison covalente sont obtenues au moyen du groupe amine libre en position amino-terminale ou du groupe carboxyle en position carboxy-terminale du résidu d'acide aminé se trouvant à l'extrémité de l'étiquette d'histidine.

4. Procédé selon l'une quelconque des revendications 1 à 3, **caractérisé en outre en ce que** les groupes réactifs dudit support ou dudit transporteur ne sont pas particulièrement sélectifs pour l'histidine mais dans lequel la sélectivité envers l'histidine est augmentée significativement, par rapport aux autres résidus, en appliquant au moins l'un des procédés suivants :
- en utilisant des conditions de réaction appropriées telles que le pH, un solvant, un excès de réactif, la cinétique, la température et similaires,
- en fusionnant l'étiquette d'histidine avec ledit peptide, ladite protéine ou ladite biomolécule à immobiliser, ou dans ceux-ci, de telle manière que ladite étiquette d'histidine soit située à l'extérieur de ladite protéine ou dudit peptide, facilitant de ce fait une interaction entre les résidus d'histidine dudit peptide ou de ladite protéine et les groupes réactifs du support ou du transporteur,
- en utilisant un nombre suffisant de résidus d'histidine pour former l'étiquette d'histidine, dans lequel ladite étiquette d'histidine contient au moins trois histidines au sein d'une extension de 6 acides aminés, en augmentant de ce fait la probabilité d'obtenir une réaction entre les groupes réactifs du support ou du transporteur et lesdits résidus d'histidine,
- en bloquant les résidus autres que l'histidine qui pourraient réagir avec les groupes réactifs dudit support ou dudit transporteur,
- en ajoutant un inhibiteur ou un substrat compétitif pour bloquer la réaction de ces acides aminés réactifs qui composent le centre actif,
- en augmentant l'hydrophobie dudit support ou dudit transporteur ou celle du micro-environnement dudit support ou dudit transporteur, en combinaison avec un abaissement du pH, en évitant de ce fait une réaction avec des résidus autres que l'histidine et en augmentant l'interaction avec des résidus d'acides aminés de ladite étiquette d'histidine, ou
- en amenant l'étiquette d'histidine tout près dudit support ou dudit transporteur au moyen de l'utilisation : d'anticorps ou de micro-protéines anti-étiquette d'histidine ; de chélateurs du Ni tels que le NTA ou l'IDA ; ou de n'importe quel autre composé se liant à une étiquette d'histidine.

5. Procédé selon l'une quelconque des revendications 1 à 4, **caractérisé en outre en ce que** les groupes réactifs dudit support ou dudit transporteur ont une sélectivité pour l'histidine et, de préférence, dans lequel les groupes réactifs sont choisis à partir des groupes suivants :
- utilisation de rose Bengale ou de bleu de méthylène pour effectuer la médiation d'une réticulation photo-oxydative entre les résidus d'histidine qui comprennent l'étiquette d'histidine et des résidus d'histidine qui sont localisés sur le transporteur ou sur la membrane,
- utilisation de pyrocarbonate de diéthyle et de dérivés de celui-ci.

6. Procédé selon l'une quelconque des revendications 1 à 5, pour immobiliser par covalence et/ou pour conjuguer par covalence des protéines, des peptides ou d'autres biomolécules sur un support ou sur un transporteur au moyen d'une étiquette d'histidine, dans lequel les groupes activés du support présentent une sélectivité envers les résidus d'histidine et dans lequel la sélectivité envers les résidus d'histidine de l'étiquette d'histidine est obtenue au moyen d'une combinaison des procédés tels qu'ils sont décrits dans les revendications 1 à 5.

7. Procédé selon l'une quelconque des revendications 1 à 3, dans lequel on laisse ladite étiquette d'histidine dudit peptide, de ladite protéine ou de ladite autre biomolécule réagir avec d'autres étiquettes d'histidine, au moyen d'une photo-réaction en présence d'oxygène et de rose Bengale, de bleu de méthylène ou de réactifs bifonctionnels, et dans lequel ladite autre étiquette d'histidine peut faire partie de protéines, de peptides ou d'autres biomolécules ou elle peut être liée à une membrane ou à un transporteur.

8. Procédé selon l'une quelconque des revendications 1 à 7, dans lequel ledit support ou ledit transporteur comprend :
- une membrane comprenant des membranes à base de styrène ou de nylon,
- d'autres protéines, peptides ou agrégats de ceux-ci,
- des micro-particules d'origine virale,
- des micro-particules de nature pplysaccharidique,
- des micro-particules protéiques,
- des biomolécules telles que des liposomes, de l'ADN, de l'ARN, des oligonucléotides, des liposomes,
- des micro-particules polymériques et des micro-particules bio-polymériques organiques de synthèse, ou
- des substrats inorganiques à base de silice.

9. Procédé selon l'une quelconque des revendications 1 à 8, dans lequel ladite étiquette d'histidine comprend n'importe quelle sorte de marqueur détectable.
